Europäisches Patentamt

European Patent Office

Office européen des brevets

(19)

(11) Publication number: **0 161 899 B1**

## EUROPEAN PATENT SPECIFICATION

(12)

(45) Date of publication of patent specification: **28.08.91**

(51) Int. Cl.5: **A61K 7/16, A61K 7/24**

(21) Application number: **85303217.5**

(22) Date of filing: **07.05.85**

The file contains technical information submitted after the application was filed and not included in this specification

(54) **Dentifrice compositions.**

(30) Priority: **09.05.84 GB 8411841**

(43) Date of publication of application:
**21.11.85 Bulletin 85/47**

(45) Publication of the grant of the patent:
**28.08.91 Bulletin 91/35**

(84) Designated Contracting States:
**AT BE CH DE FR GB IT LI NL SE**

(56) References cited:
**US-A- 4 022 880**

(73) Proprietor: **UNILEVER PLC**
**Unilever House Blackfriars P.O. Box 68**
**London EC4P 4BQ(GB)**

(84) Designated Contracting States:
**GB**

Proprietor: **UNILEVER NV**
**Burgemeester s'Jacobplein 1 P.O. Box 760**
**NL-3000 DK Rotterdam(NL)**

(84) Designated Contracting States:
**BE CH DE FR IT LI NL SE AT**

(72) Inventor: **Saxton, Charles Allan**
**8 Thornton Crescent Gayton**
**Wirral Merseyside(GB)**

(74) Representative: **van Gent, Jan Paulus et al**
**Unilever N.V., Patent Division Postbus 137**
**NL-3130 AC Vlaardingen(NL)**

EP 0 161 899 B1

## Description

This invention relates to dentifrice compositions, more particularly to toothpastes having an anti-plaque activity.

It is now established that there is a relationship between dental plaque and gingival inflammation. At present, mechanical cleaning by toothbrushing is the most widely used method of removing plaque. However, the relatively short period of brushing commonly practised is insufficient to achieve adequate removal of plaque especially from areas least accessible to brushing.

The dental literature over the last 25 years is replete with publications concerned with the use of organic antimicrobial agents to combat dental plaque. Most work has concentrated on the use of cationic agents because these are substantive to oral tissues and are therefore retained in the mouth. It is believed that their activity is due to their being adsorbed onto oral surfaces and gradually released over a period of time (Journal of Clinical Periodontology 1980: 7 431-442). Unless an active antimicrobial agent is adsorbed onto oral surfaces the oral bacteria in the mouth rapidly recover and no significant reduction in plaque growth would be expected (British Dental Journal March 1984, 175-178). However, the successful formulation of a cationic antimicrobial agent into a commercially acceptable toothpaste has not yet been achieved and this is at least in part due to the incompatibility of cationic antimicrobial agents with common toothpaste ingredients. In addition, cationics, especially chlorhexidine which has been extensively investigated, have the further disadvantage of causing tooth staining as well as having a long-lasting bitter taste. Many cationics also cause irritation of the oral tissues. Attempts to overcome the tooth staining problem are the subject of many patents. We believe that there is to date no commercial toothpaste containing an organic cationic antimicrobial agent which is recognised as having a significant anti-plaque benefit.

Although there are references in the literature to attempts to use antimicrobial agents other than cationic compounds for providing an improvement in oral health it is generally considered that the oral substantivity of these agents is not sufficient to provide a significant antiplaque benefit and they are in any case considered unattractive because of their generally poor water-solubility. Hitherto it has been the general belief that it is necessary for the active agent of a dentifrice to be in solution in the aqueous phase of the toothpaste.

In Us- 4 022 880 the co-action of zinc ions and an anti-bacterial agent is exposed. From the examples can be deduced that a) if one wants to use zinc citrate, at least 10% should be used, and b) if one wants to use Triclosan, it should be used in combination with a soluble zinc salt such as zinc acetate.

Toothpastes are highly complex systems and it is difficult to formulate an effective anti-plaque toothpaste which is acceptable to the consumer, especially from the point of view of taste and freedom from staining. We have now developed a toothpaste comprising an effective anti plaque system which has a high degree of consumer acceptance.

According to the invention there is provided a dentifrice composition effective to inhibit the growth of dental plaque comprising

a) an abrasive agent consisting of alumina trihydrate or silica or mixture thereof

b) 0.05 to 2%, preferably 0.2 to 1.5%, by weight zinc citrate trihydrate, and

c) 0.1 to 1% by weight 2'4,4'-trichloro-2-hydroxy-diphenyl ether (hereinafter referred to as Triclosan).

The toothpaste of the invention combines in single formulation a number of highly desirable qualities. The toothpaste is non-staining, has low astringency and therefore easily flavoured, the active ingredients have good compatibility with the abrasive and other toothpaste components, and the toothpaste has good activity against dental plaque. This combination of properties is not possessed by any commercially available toothpaste.

Triclosan is substantially insoluble in water whereas cationics in general have much higher water solubilities.

The dentifrice composition of the invention comprises a particulate hydrated alumina or silica abrasive or mixture thereof. The particle size of the abrasive agent will usually be in the range 2 to 20 microns as is customary. Suitable grades of alpha-alumina trihydrate are sold under the name of BACO by BA Chemicals of Great Britain and under the name MARTINAL by Martinswerke GmbH of Germany. Preferred silica abrasives are the well-known silica xerogels, for example GASIL 200 (sold by Crosfield Chemicals, Great Britain), and SYLOID 63 (sold by Grace Corporation USA), and precipitated silicas, for example ZEO 49 (sold by the Huber Corporation USA). The amount of abrasive agent employed will usually be between 5 and 60% by weight of the dentifrice composition.

The dentifrice composition may also comprise other ingredients well known to those skilled in the art. Thus the dentifrice will contain a humectant liquid, surfactant and thickening agent. Suitable humectants are glycerol, sorbitol and propylene glycol. Anionic surfactants are usually used especially sodium lauryl sulphate or a mixture thereof with sodium dodecylbenzenesulphonate, for example in the weight ratio of 4:1 to 1:4. Other surfactants can be used such as sodium lauroyl sarcosinate. Suitable binders or thickeners for use in dentifrice compositions are known to those skilled in the art. Commonly used are sodium carboxymethylcellulose and xanthan gum. For flavouring dentifrices peppermint and spearmint oils are commonly used, although a wide variety of other oils also find application. Apart from the above generally standard ingredients, a number of optional ingredients may also be included, especially fluoride, such as sodium monoflurophosphate, opacifying agent, eg titanium dioxide, preservative, sweetening agent and a pH-adjusting agent.

Typical dentifrice compositions in accordance with this invention comprise 10 to 70% by weight humectant, 0.3 to 3% surfactant, 0.1 to 2% binding agent.

The following Examples illustrate the invention. Percentages are by weight.

## Example 1

A toothpaste was made having the following composition.

| Ingredient | % |
|---|---|
| Alumina trihydrate | 50.000 |
| Sorbitol syrup (70% solution) | 27.000 |
| Sodium lauryl sulphate | 1.875 |
| Sodium dodecylbenzenesulphonate | 0.625 |
| Sodium carboxymethylcellulose | 0.800 |
| Zinc citrate trihydrate | 0.500 |
| Triclosan | 0.200 |
| Sodium monofluorophosphate | 0.850 |
| Flavour oil | 1.200 |
| Sodium saccharinate | 0.180 |
| Formalin BP | 0.040 |
| Water | to 100.000 |

The effectiveness of the dentifrice compositions of this invention in inhibiting the growth of plaque on the teeth was determined by following a standard procedure for the measurement of plaque growth. The methodology of measuring plaque growth is that according to Harrap as described in J.Clin.Periodontol., 1974, 1, 166-174 which gives a procedure for assessing the amount of plaque on the teeth adjacent to the gingival margin. The procedure is as follows:

During the late afternoon each subject brushes his teeth with a simple, non-active paste (having a composition as given hereinafter) for an unspecified period of time to remove as much plaque as possible. This is immediately followed by brushing for one minute with 1.5g of the allocated test paste. Residual paste is removed by rinsing the mouth with water and any remaining plaque disclosed by painting the teeth with an aqueous solution of Erythrosin (0.5% w/w) using a soft camel hair brush. Excess dye is removed by rinsing with water and the amount of plaque assessed and recorded for each of 16 teeth (numbers 3 to 6 for each quadrant). The recorded plaque is designated $P_0$.

No further oral hygiene is permitted for 18 hours after which time each subject rinses his mouth with water to remove food debris and viscous saliva. Plaque assessment is then carried out as before and recorded ($P_{18}$). The values of $P_{18}-P_0$ for each tooth are averaged to give a $P_{18}-P_0$ value per mouth. The mean of the values obtained for the subjects in the test is the plaque growth value. Panels of at least 12 subjects are used. The plaque growth value for a toothpaste without active ingredients is usually in the range 22 to 26.

3

The composition of the simple, non-active toothpaste referred to above was the following:-

| Ingredient | % |
|---|---|
| Alumina trihydrate | 50.00 |
| Glycerin | 27.00 |
| Hydroxyethylcellulose | 0.95 |
| Titanium dioxide | 0.50 |
| Water | to 100.00 |

The plaque growth value for the toothpaste of Example 1 was 16.4 whereas that for the toothpaste without the Triclosan was 18.8.

The consumer acceptability of the toothpaste of this Example was compared with that of the product without the Triclosan in a panel test involving panels of 150 people. The tests showed that the toothpastes were equally acceptable. Thus the acceptability of the toothpaste was not reduced by the inclusion of the antimicrobial agent Triclosan. Analysis of the data showed a trend towards a preference for products containing Triclosan and zinc citrate in respect of consumer attributes compared to the product with zinc citrate only.

Thus the toothpaste of the invention has highly desirable overall properties. They do not cause any staining of the teeth. The active ingredients are highly compatible with the abrasive and other components of the toothpaste. There was also a lack of irritation of the oral tissues.

Example 2

A toothpaste was made as in Example 1 except that the amount of zinc citrate was 1% and the amount of Triclosan was 0.5%.

Example 3

A toothpaste was made as in Example 1 except that the amount of zinc citrate was 1% and the amount of Triclosan was 0.2%.

Example 4

A toothpaste was made as in Example 1 except that the amount of zinc citrate was 0.5% and the amount of Triclosan was 0.5%.

The toothpastes of Example 2 to 4 were tested using the same protocol as for Example 1. Each of the products of Examples 2 to 4 gave the same results as for Example 1.

Examples 5 and 6 are further Examples of toothpastes according to this invention.

Example 5

A toothpaste was made except that the sodium lauryl sulphate was present at a level of 1.6% and the sodium dodecylsulphonate was omitted. This had a plaque growth value of 15.3.

Example 6

A toothpaste having the following composition was made.

| Ingredients | % |
|---|---|
| Silica xerogel | 10.00 |
| Precipitated silica | 8.50 |
| Sorbitol syrup (70%) | 69.50 |
| Sodium lauryl sulphate | 1.70 |
| Sodium dodecylbenzene sulphonate | 0.50 |
| Sodium carboxymethylcellulose | 0.50 |
| Polyethylene glycol (1500) | 5.00 |
| Ethanol | 1.80 |
| Zinc citrate trihydrate | 0.50 |
| Triclosan | 0.20 |
| Sodium monofluorophosphate | 0.75 |
| Flavour oil | 0.77 |
| Sodium saccharin | 0.30 |
| Water | to 100.00 |

This had a plaque growth value of 16.3.

**Claims**

1. A dentifrice composition effective to inhibit the growth of dental plaque comprising
   a) an abrasive agent consisting of alumina trihydrate or silica or mixture thereof
   b) 0.05 to 2% by weight zinc citrate trihydrate, and
   c) 0.1 to 1% by weight Triclosan.

2. A dentifrice composition as claimed in Claim 1, comprising 30 to 60% by weight alumina trihydrate, 0.2 to 1.5% by weight zinc citrate and 0.1 to 1% Triclosan.

3. A dentifrice composition as claimed in Claim 1, wherein the abrasive is a silica xerogel.

4. A dentifrice composition as claimed in Claim 1, wherein the abrasive is precipitated silica.

**Revendications**

1. Une composition dentifrice efficace pour inhiber la croissance de la plaque dentaire, comportant :
   a) un agent abrasif constitué d'alumine trihydratée ou de silice ou d'un mélange de celles-ci ;
   b) 0,05 à 2 % en poids de citrate trihydraté de zinc ; et
   c) 0,1 à 1 % en poids de Triclosan.

2. Une composition dentifrice telle que revendiquée dans la revendication 1, comportant 30 à 60 % en poids d'alumine trihydratée, 0,2 à 1,5 % en poids de citrate de zinc et 0,1 à 1 % de Triclosan.

3. Une composition dentifrice telle que revendiquée dans la revendication 1,dans laquelle l'abrasif est un xérogel de silice.

4. Une composition dentifrice telle que revendiquée dans la revendication 1, dans laquelle l'abrasif est une silice précipitée.

**Patentansprüche**

5

1. Zahnpflegemittel-Zusammensetzung, die das Wachstum von Zahnbelägen wirksam hemmt, umfassend
   a) ein Schleifmittel, bestehend aus Aluminiumoxid-Trihydrat oder Siliciumoxid oder einer Mischung davon,
   b) 0,05 bis 2 Gew.-% Zinkcitrat-Trihydrat und
   c) 0,1 bis 1 Gew.-% Triclosan.

2. Zahnpflegemittel-Zusammensetzung nach Anspruch 1, umfassend 30 bis 60 Gew.-% Aluminiumoxid-Trihydrat, 0,2 bis 1,5 Gew.-% Zinkcitrat und 0,1 bis 1 % Triclosan.

3. Zahnpflegemittel-Zusammensetzung nach Anspruch 1, wobei das Schleifmittel ein Siliciumoxid-Xerogel ist.

4. Zahnpflegemittel-Zusammensetzung nach Anspruch 1, wobei das Schleifmittel gefälltes Siliciumoxid ist.